# EUROPEAN PATENT APPLICATION

(11) **EP 3 017 803 A1**
(43) Date of publication of application: **11.05.2016**
(21) Application number: 14805771.4
(22) Date of filing: 19.06.2014
(51) Int. Cl.: A61H 33/02, A61K 8/19, A61K 33/00, A61P 17/00, A61Q 19/08

(54) **DEVICE FOR MANUFACTURING GAS FOR PERCUTANEOUS ABSORPTION, METHOD FOR MANUFACTURING PERCUTANEOUS ABSORPTION GAS, AND PERCUTANEOUS ABSORPTION GAS**

(30) Priority: 04.07.2013 JP 2013140896
(71) Applicant: Neochemir Inc., Kobe-shi Hyogo 651-0087 (JP)
(72) Inventor: TANAKA, Masaya, Kobe-shi Hyogo 651-0087 (JP)
(74) Representative: Sajda, Wolf E.
(86) International application number: PCT/JP2014/066243
(87) International publication number: WO 2015/001964

(57) **Abstract**

An apparatus for preparing a gas for transdermal absorption is provided with: a carbon dioxide supplying unit (1) that supplies gaseous carbon dioxide; a solvent vapor generating unit (2) that generates vapor of a solvent (20), being capable of dissolving the carbon dioxide and vaporable; and a generating unit of a gas for transdermal absorption that generates a gas for transdermal absorption containing the gaseous carbon dioxide-solvent complex by mixing the supplied carbon dioxide and the generated solvent vapor, and is characterized by being further provided with a floating liquid drop removing unit (4) that removes a floating liquid drop of the solvent (20) from the generated gas for transdermal absorption.

## Description

### TECHNICAL FIELD

The present invention relates to an apparatus and a method for preparing a gas for transdermal absorption for making carbon dioxide to be transdermally absorbed into an animal or a human, and to a gas for transdermal absorption prepared by the apparatus and the method.

### BACKGROUND ART

It has already been known that various medical and cosmetic symptoms are improved when carbon dioxide is taken into the human body by transdermal absorption (see Patent Document 1). The main mechanisms of action of the carbon dioxide in these cases are a vasodilating action and atissue oxygen partial pressure increasing action (see Non-Patent Document 1). Therefore, the effects of the carbon dioxide can be predicted by the presence or absence and degree of the redness of the skin that reflects the vasodilating action.

Carbon dioxide is hardly transdermally absorbed by itself. This is clear from the fact that the effects of carbon dioxide such as the vasodilating action are not obtained even though carbon dioxide gas is blown to the skin. In order for carbon dioxide to show the vasodilating action and the like, carbon dioxide must be in a state of being dissolved in a solvent such as water, that is, must be dissolved carbon dioxide (Patent Document 2).

That is, in order to obtain the effects of carbon dioxide, it is necessary to apply a carbon dioxide solution to the skin. However, the solubility of carbon dioxide is, for example when the solvent is water, as low as approximately 0.1 %. For example, carbonated water immediately trickles down when applied to the skin, and does not exert the effects of carbon dioxide.

To solve the above-mentioned problem, the present inventor has already proposed various external carbon dioxide agents where carbon dioxide is continuously generated in a viscous composition adhering to the skin (see Patent Documents 3 to 7). Further, the present inventor has proposed a device comprising: a sealing enclosure member capable of sealing off a body surface from the outside air; a supply unit for supplying carbon dioxide into the inside of the sealing enclosure member; and an absorption aid that assists transdermal absorption of the carbon dioxide inside the sealing enclosure member for a wide skin area transdermal absorption at a time. (see Patent Document 8).

Further, methods of making carbon dioxide to be transdermally absorbed by using gaseous carbon dioxide are disclosed in Patent Documents 9 to 19.

Moreover, it is known that carbon dioxide and the vapor of a solvent (for example, water) form a reversibly bound complex (see Non-Patent Documents 2 and 3). This complex can be called a "carbon dioxide-solvent complex".

### Prior Art Documents

### Patent Document

Patent Document 1 : JP 2000-319 187 A
Patent Document 2 : JP 60-215 606 A
Patent Document 3 : WO 2002/080941 A1
Patent Document 4 : WO 2006/080398 A1
Patent Document 5 : WO 2003/057228 A1
Patent Document 6 : WO 2005/016290 A1
Patent Document 7 : WO 2004/004745 A1
Patent Document 8 : WO 2004/002393 A1
Patent Document 9 : JP 07-171 189 A
Patent Document 10 : JP 2010-029 265 A
Patent Document 11 : JP 2010-029 267 A
Patent Document 12 : JP 2007-252 871 A
Patent Document 13 : JP 2006-034 613 A
Patent Document 14 : JP 2006-263 253 A
Patent Document 15 : JP 2006-034 614 A
Patent Document 16 : JP 2009-011 695 A
Patent Document 17 : JP 2009-183 625 A
Patent Document 18 : JP 2009-183 626 A
Patent Document 19 : JP 2009-254 726 A

### Non-Patent Document

Non-Patent Document 1 : Toshiki HIYOSHI, Jinko tansansen yokuzai ni yoru jokuso chiryo ni tsuite (Regarding Treatment of Bedsore by Bath Agent of Artificial Carbonated Spring), Sogo Riha 17(8), 605-9, 1989
Non-Patent Document 2 : K.I.Peterson, W.Klemperer, J.Chem.Phys. 1984, 80, 2439.
Non-Patent Document 3 : P.A.Block, M.D.Marshall, L.G.Pedersen, R.E.Miller, J.Chem.Phys. 1992, 96, 7321.

### SUMMARY OF INVENTION

### PROBLEM TO BE SOLVED BY THE INVENTION

An object of the present invention is to obtain a gas for transdermal absorption that can be easily applied even to the skin covered with clothing or the like without the clothing or the skin getting wet and further, is capable of exerting the effects of being transdermally absorbed carbon dioxide remarkably.

### MEANS FOR SOLVING THE PROBLEM

An apparatus for preparing a gas for transdermal absorption of the present invention is an apparatus for preparing a gas for transdermal absorption containing a gaseous carbon dioxide-solvent complex, which is provided with: a carbon dioxide supplying unit that supplies gaseous carbon dioxide; a solvent vapor generating unit that generates vapor of a solvent, being capable of dissolving carbon dioxide and vaporable; and a generating unit of a gas for transdermal absorption that generates a gas for transdermal absorption containing the gaseous carbon dioxide-solvent complex by mixing the supplied carbon dioxide and the generated solvent vapor, and is characterized by (a) being further provided with a floating liquid drop removing unit that removes a floating liquid drop of the solvent from the generated gas for transdermal absorption or (b) including a contamination preventing means for preventing contamination of the floating liquid drop of the solvent into the generated gas for transdermal absorption.

A method for preparing a gas for transdermal absorption of the present invention is a method for preparing a gas for transdermal absorption containing a gaseous carbon dioxide-solvent complex, which is provided with: a carbon dioxide supplying process for supplying gaseous carbon dioxide; a solvent vapor generating process for generating vapor of a solvent, being capable of dissolving carbon dioxide and vaporable; and a gas for transdermal absorption generating process for generating a gas for transdermal absorption containing the gaseous carbon dioxide-solvent complex prepared by mixing the supplied carbon dioxide and the generated solvent vapor, and is characterized by (a) being further provided with a floating liquid drop removing process for removing a floating liquid drop of the solvent from the generated gas for transdermal absorption or (b) including a contamination preventing process for preventing contamination of the floating liquid drop of the solvent into the generated gas for transdermal absorption.

A gas for transdermal absorption of the present invention is characterized by being obtained by using the above-described transdermal absorption gas preparing apparatus or by the above-described transdermal absorption gas preparing method.

The present inventor calculated that the carbon dioxide-solvent complex will be transdermally absorbed similarly to the dissolved carbon dioxide since it has a molecular structure equivalent to the dissolved carbon dioxide in that a carbon dioxide molecule and a solvent molecule are reversibly bonded. On the other hand, if containing a large amount of floating liquid drops of the solvent, the gas for transdermal absorption containing the carbon dioxide-solvent complex becomes dissolved carbon dioxide by the carbon dioxide being absorbed into the floating liquid drops (see Patent Documents 9 to 19).

The present inventor found as a result of earnest research that the gas for transdermal absorption containing the carbon dioxide-solvent complex can powerfully deliver the effect of transdermally absorbed carbon dioxide when it contains no floating liquid drops of the solvent, and completed the present invention.

In the present invention, the floating liquid drops of the solvent indicates fine particles of the solvent that are floating in the air and whose sizes are approximately not more than 100 µm in diameter. The vapor of the solvent indicates a gas where solvent molecules are basically present discretely one by one.

### EFFECTS OF THE INVENTION

According to the apparatus for preparing a gas for transdermal absorption of the present invention, since the floating liquid drops of the solvent are removed by the floating liquid drop removing unit from the gas for transdermal absorption obtained by the generating unit of the gas for transdermal absorption, the gas for transdermal absorption contains no floating liquid drops.

Alternatively, according to the apparatus for preparing a gas for transdermal absorption of the present invention, since the contamination of floating liquid drops of the solvent into the gas for transdermal absorption obtained by the generating unit of the gas for transdermal absorption is prevented by the contamination preventing means, the obtained gas for transdermal absorption contains no floating liquid drops. That is, according to the apparatus for preparing a gas for transdermal absorption of the present invention, a gas for transdermal absorption containing no floating liquid drops of the solvent can be obtained.

According to the method for preparing a gas for transdermal absorption of the present invention, since the floating liquid drops of the solvent are removed by the floating liquid drop removing process from the gas for transdermal absorption generated through the generating process of the gas for transdermal absorption, the gas for transdermal absorption contains no floating liquid drops.

Alternatively, according to the method for preparing a gas for transdermal absorption of the present invention, since the contamination of floating liquid drops of the solvent into the gas for transdermal absorption generated at the transdermal absorption gas generating process is prevented by the contamination preventing process, the gas for transdermal absorption contains no floating liquid drops. That is, according to the method for preparing a gas for transdermal absorption of the present invention, a gas for transdermal absorption containing no floating liquid drops of the solvent can be obtained.

According to the gas for transdermal absorption of the present invention, since no floating liquid drops of the solvent are contained, it can be accomplished that highly concentrated carbon dioxide is transdermally absorbed without depending on the solubility of carbon dioxide to the solvent, so that the following effects can be delivered:
(1) The effects of transdermally absorbed carbon dioxide can be improved easily and significantly.
(2) It can be accomplished that carbon dioxide is transdermally absorbed with ease from a wide area of the skin.
(3) It can be accomplished that carbon dioxide is transdermally absorbed without the clothing and the skin getting wet.
(4) It can be accomplished that carbon dioxide is transdermally absorbed with ease also from the skin covered with clothing or the like.

### BRIEF DESCRIPTION OF THE DRAWINGS

FIG. 1 is a schematic structure diagram showing a first preparing apparatus of the present invention.
FIG. 2 is a schematic structure diagram showing a second preparing apparatus of the present invention.
FIG. 3 is a schematic structure diagram showing a third preparing apparatus of the present invention.
FIG. 4 is a side cross-sectional schematic diagram of the preparing apparatus used in Example 1.
FIG. 5 is a partially omitted plan view of the apparatus of FIG. 4.
FIG. 6 is a side cross-sectional schematic diagram of the preparing apparatus used in Examples 2 to 10.
FIG. 7 is a side cross-sectional schematic diagram of the preparing apparatus used in Example 11.
FIG. 8 is a partially omitted plan view of the apparatus of FIG. 7.

### MODE FOR CARRYING OUT THE INVENTION

### Preparing Apparatus

An apparatus for preparing a gas for transdermal absorption of the present invention can be broadly divided into two types. A first apparatus is provided with a floating liquid drop removing unit that removes floating liquid drops of the solvent from the generated gas for transdermal absorption. A second apparatus includes contamination preventing means for preventing the contamination of floating liquid drops of the solvent into the generated gas for transdermal absorption.

The first apparatus for preparing a gas for transdermal absorption is an apparatus for preparing a gas for transdermal absorption containing a gaseous carbon dioxide-solvent complex, and is characterized by being provided with: a carbon dioxide supplier that supplies gaseous carbon dioxide; a solvent vapor generating unit that generates the vapor of a solvent, being capable of dissolving carbon dioxide and vaporable; and a generating unit of a gas for transdermal absorption that generates a gas for transdermal absorption containing a gaseous carbon dioxide-solvent complex by mixing the supplied carbon dioxide and the generated solvent vapor, and by being further provided with the floating liquid drop removing unit that removes the floating liquid drops of the solvent from the generated gas for transdermal absorption.

The floating liquid drop removing unit can be broadly divided into two types. A first floating liquid drop removing unit has heating means for evaporating the floating liquid drops contained in the gas for transdermal absorption. A second floating liquid drop removing unit has absorbing means for absorbing the floating liquid drops contained in the gas for transdermal absorption. The absorbing means is a chemical absorbent or a physical absorbent.

The second apparatus for preparing a gas for transdermal absorption is an apparatus for preparing a gas for transdermal absorption containing a gaseous carbon dioxide-solvent complex, and is characterized by being provided with: a carbon dioxide supplier that supplies gaseous carbon dioxide; a solvent vapor generator generating unit that generates the vapor of a solvent, being capable of dissolving carbon dioxide and vaporable; and a generating unit of a gas for transdermal absorption that generates a gas for transdermal absorption containing a gaseous carbon dioxide-solvent complex by mixing the supplied carbon dioxide and the generated solvent vapor, and by including contamination preventing means for preventing the contamination of floating liquid drops of the solvent into the generated gas for transdermal absorption.

The contamination preventing means is a carbon dioxide supplying unit structured so as to supply carbon dioxide containing no floating liquid drops and a solvent vapor generating unit structured so as to generate solvent vapor containing no floating liquid drops. Specifically, the carbon dioxide supplying unit is composed so as to supply liquefied carbon dioxide after vaporizing it. Or the carbon dioxide supplying unit has heating means for evaporating the floating liquid drops contained in the carbon dioxide.

Or the carbon dioxide supplying unit has absorbing means for absorbing the floating liquid drops contained in the carbon dioxide. Moreover, the solvent vapor generating unit has heating means for heating the solvent so that the vapor pressure thereof is not more than the saturated vapor pressure at that temperature. Or the solvent vapor generating unit has absorbing means for absorbing the floating liquid drops contained in the solvent vapor.

### Preparing Method

The method for preparing a gas for transdermal absorption of the present invention can be broadly divided into two types. A first method has a floating liquid drop removing process for removing the floating liquid drops of the solvent from the generated gas for transdermal absorption. A second method includes a contamination preventing process for preventing the contamination of floating liquid drops of the solvent into the generated gas for transdermal absorption.

The first method for preparing a gas for transdermal absorption is a method for preparing a gas for transdermal absorption containing a gaseous carbon dioxide-solvent complex, and is characterized by having: a carbon dioxide supplying process for supplying gaseous carbon dioxide; a solvent vapor generating process for generating the vapor of a solvent, being capable of dissolving carbon dioxide and vaporable; and a gas for transdermal absorption generating process for generating a gas for transdermal absorption containing a gaseous carbon dioxide-solvent complex by mixing the supplied carbon dioxide and the generated solvent vapor, and by further having the floating liquid drop removing process for removing the floating liquid drops of the solvent from the generated gas for transdermal absorption.

The floating liquid drop removing process can be broadly divided into two types. A first floating liquid drop removing process has a heating process for evaporating the floating liquid drops contained in the gas for transdermal absorption. A second floating liquid drop removing process has an absorbing process for absorbing the floating liquid drops contained in the gas for transdermal absorption. The absorbing process is carried out by using a chemical absorbent or a physical absorbent.

The second method for preparing a gas for transdermal absorption is a method for preparing a gas for transdermal absorption containing a gaseous carbon dioxide-solvent complex, and is characterized by having: a carbon dioxide supplying process for supplying gaseous carbon dioxide; a solvent vapor generating process for generating the vapor of a solvent, being capable of dissolving carbon dioxide and vaporable; and a gas for transdermal absorption generating process for generating a gas for transdermal absorption containing a gaseous carbon dioxide-solvent complex by mixing the supplied carbon dioxide and the generated solvent vapor, and by including a contamination preventing process for preventing the contamination of floating liquid drops of the solvent into the generated gas for transdermal absorption.

The contamination preventing process is a carbon dioxide supplying process for supplying carbon dioxide containing no floating liquid drops and a solvent vapor generating process for generating solvent vapor containing no floating liquid drops. Specifically, the carbon dioxide supplying process supplies liquefied carbon dioxide after vaporizing it. Or the carbon dioxide supplying process has a heating process for evaporating the floating liquid drops contained in the carbon dioxide. Or the carbon dioxide supplying step has an absorbing step of absorbing the floating liquid drops contained in the carbon dioxide.

Moreover, the solvent vapor generating process has a heating process for heating the solvent so that the vapor pressure thereof is not more than the saturated vapor pressure at that temperature. Or the solvent vapor generating process has an absorbing process for absorbing the floating liquid drops contained in the solvent vapor. The absorbing process is carried out by using a chemical absorbent or a physical absorbent.

In any of these methods, although the floating liquid drops must be as few as possible in the obtained gas for transdermal absorption, it is unnecessary that the floating liquid drops be completely removed and it is necessary only that the proportion of the dissolved carbon dioxide that is formed by absorbing carbon dioxide by the floating liquid drops be small. To prevent the generation of floating liquid drops, it is desirable that the gas for transdermal absorption be (a) always maintained at a temperature not less than the room temperature, that (b) the vapor pressure of the solvent is not more than the saturated vapor pressure and that (c) fine solid particles, such as dust, promoting the condensation of the solvent vapor are few. If the floating liquid drops are too many, even if the carbon dioxide concentration, the solvent vapor pressure and the temperature are the same, the blood flow increasing action is reduced.

### Details

### Gas for Transdermal Absorption

For the obtained gas for transdermal absorption, it is necessary only that not less than 15 vol% of carbon dioxide be contained and the vapor pressure of the solvent be not less than 30 % of the saturated vapor pressure, and it is preferable to have a temperature of not less than 25 °C. The carbon dioxide content of the gas for transdermal absorption is more preferably not less than 30 vol%, and is most preferably not less than 50 vol%. The vapor pressure of the solvent is more preferably not less than 50 % of the saturated vapor pressure, and is most preferably not less than 70 % thereof. The present inventor has elucidated that the transdermal absorption efficiency of carbon dioxide decreases when the skin temperature is not more than 25 °C.

### Floating Liquid Drop Checking Method

The following methods (a), (b) or (c) may be adopted. However, the present invention is not limited thereto.
(a) Observation is performed by using a fine particle measuring instrument or the like under a condition where dust and the like are few.
(b) The presence or absence of a Tyndall phenomenon is observed with the naked eye. When no floating liquid drops are contained, no Tyndall phenomenon is recognized. Specifically, in a dark airtight container, a bundle of light is applied to the object, and the presence or absence of a light path is observed. When no light path is recognized, it can be determined that there are no floating liquid drops.
(c) A sheet made of a material having low thermal conductivity such as paper is exposed to the object. If there are floating liquid drops, the floating liquid drops are absorbed by the sheet to make stains. A sheet made of a material having high thermal conductivity such as a metal is not preferable because even if there are no floating liquid drops, when the vapor of the solvent touches it, it deprives the vapor of heat and this causes the solvent to be condensed to make stains on the sheet like the floating liquid drops.

### Quantity Determination of Floating Liquid Drops

As a method of determining the quantity of the floating liquid drops, a method may be adopted of exposing a sheet made of a low thermal conductivity material that absorbs floating liquid drops (for example, filter paper), to the object for a predetermined time and measuring the quantity of absorption. Specifically, it is as follows:
(1) First, the total mass of the filter paper and a non-porous aluminum pouch is measured to obtain a first measurement value.
(2) Next, after exposed to the object for the predetermined time, the filter paper is immediately put into the aluminum pouch and sealed hermetically. Then, the total mass thereof is measured to obtain a second measurement value. Since the solvent that forms floating liquid drops is vaporable, the floating liquid drops absorbed by the filter paper are vaporized from the filter paper with time. Therefore, to precisely measure the absorption quantity of the floating liquid drops, the filter paper must be put into the aluminum pouch and hermetically sealed "immediately" after the exposure.
(3) On the other hand, since the filter paper also absorbs the vapor of the solvent (water vapor when the solvent is water), after exposed to the vapor of the solvent having a humidity at the same temperature as the object for a predetermined time, the filter paper is immediately put into an aluminum pouch and sealed hermetically. Then, the total mass thereof is measured to obtain a third measurement value.
(4) Then, the difference between the third measurement value and the first measurement value is subtracted from the difference between the second measurement value and the first measurement value. Thereby, the mass of the floating liquid drops absorbed by the filter paper is obtained.

### Solvent

As the solvent, any may be used without a specific limitation as long as it is capable of dissolving gaseous carbon dioxide, is liquid at room temperature and is vaporable. Specifically, an inorganic solvent or an organic solvent may be used.

As the inorganic solvent, water is preferable. As the water, tap water, distilled water, membrane filtrate water, ion-exchanged water, electrolyzed water or the like may be used and there is no specific limitation.

As the organic solvent, the following may be used: monohydric alcohol such as methanol, ethanol, n-propanol, isopropanol, n-butyl alcohol, isobutyl alcohol, sec-butyl alcohol, or tert-butyl alcohol; dihydric alcohol such as 1, 3-butylene glycol, ethylene glycol, diethylene glycol, or propylene glycol; and glycol ether such as 2-phenoxyethanol.

As the solvent, water is most preferable. When water is used as the solvent, although not specifically limited, it is preferable that the pH thereof be less than 7. This is because when the water is alkaline, carbon dioxide is consumed by being converted into bicarbonate ion, carbonate ion or the like in the water and this reduces the generation of the carbon dioxide-solvent complex. As the method of making the pH of the water less than 7, while just dissolving carbon dioxide in water will do, an acid substance may be dissolved in water. As the acid substance, organic acid or inorganic acid may be used without a specific limitation. Alternatively, acid electrolyzed water may be used as the water.

### Carbon Dioxide

As the carbon dioxide source that generates carbon dioxide, there is no specific limitation, and liquefied carbon dioxide, dry ice or the like may be used. Moreover, carbon dioxide may be generated by the reaction, for example, between acid and carbonate. Among these, liquefied carbon dioxide is preferable because it contains few impurities such as moisture and contains hardly any floating liquid drops. It is preferable that the liquefied carbon dioxide be used after pressure-reduced by using a pressure regulator. Moreover, it is preferable that the liquefied carbon dioxide be used after heated since low-temperature carbon dioxide gas emerges from the pressure regulator.

### Absorbent

As the absorbent, a chemical absorbent and a physical absorbent may be used. As the chemical absorbent, calcium chloride, magnesium sulfate, diphosphorus pentoxide, concentrated sulfuric acid, magnesium perchlorate or the like may be used. As the physical absorbent, zeolite, silica gel, aluminum oxide, activated carbon, cotton, a molecular sieve or the like may be used. These absorbents can be used similarly when the solvent is alcohol or oil as well as when the solvent is water. It is desirable to use an absorbent adapted to the physical properties of the solvent. More specifically, for example, a solvent and an absorbent that do not chemically react with each other are combined.

### Carbon Dioxide Containing No Floating Liquid Drops

Carbon dioxide containing no floating liquid drops can be obtained in the following manner:
(a) Liquefied carbon dioxide is pressure-reduced by using a pressure regulator. Since liquefied carbon dioxide is very pure carbon dioxide containing no moisture or the like, it contains hardly any floating liquid drops when liquefied.
(b) Floating liquid drops are removed from carbon dioxide containing floating liquid drops. Examples of this floating liquid drop removing method include an absorption method and a heating method.

### (b-1) Absorption method

For example, when so-called dry ice is used, the carbon dioxide generated from dry ice generates floating liquid drops (white smoke) since it cools water vapor in the air and condenses it. Therefore, the carbon dioxide generated from the dry ice is exposed to the absorbent. By doing this, the floating liquid drops contained in the carbon dioxide are removed by being absorbed by the absorbent. As the absorbent, the above-mentioned ones may be used. Carbon dioxide generated by microbial fermentation may be used by processing similarly to in the case of dry ice.

### (b-2) Heating method

Carbon dioxide containing floating liquid drops is heated to evaporate the floating liquid drops. As specific heating methods, (i) a method of applying heat rays such as infrared rays or far infrared rays to the carbon dioxide containing floating liquid drops, (ii) a method of bringing the carbon dioxide containing floating liquid drops into contact with a high-temperature member such as an electric heater, or the like may be adopted. The heating method is not limited to the above-mentioned methods as long as it is not a method causing chemical change on the carbon dioxide or the solvent.

Although the heating temperature may be any that is not less than room temperature, if it is too high, the obtained gas for transdermal absorption burns the human body and the like; therefore, it is preferably set so that the temperature of the gas for transdermal absorption obtained after heating is not more than 100 °C, further, not more than 50 °C. According to a study by the present inventor, it has been found that the transdermal absorption of carbon dioxide is difficult when the skin temperature is less than 25 °C. Therefore, it is preferable to set the heating temperature so that the temperature of the gas for transdermal absorption obtained after heating is not less than 25 °C.

### Solvent Vapor Containing No Floating Liquid Drops

The solvent vapor containing no floating liquid drops can be obtained in the following manner:
(a) The solvent is heated so that the vapor pressure is not more than the saturated vapor pressure at that temperature. It is preferable that the temperature of the heating source be not less than the boiling point of the solvent in order to efficiently generate solvent vapor. Although it is as a matter of course that the upper limit of the temperature of the heating source is a temperature at which the solvent does not decompose, temperatures up to a temperature approximately 50 °C higher than the boiling point of the solvent are allowable. When the solvent is water, it is preferable to heat the solvent to not less than 100 °C and not more than 150 °C.
   The vapor pressure of the obtained solvent vapor must be not more than the saturated vapor pressure of the solvent at that temperature. When the solvent is water, the humidity may be any that is not more than 100 %. Even when the vapor pressure is not more than the saturated vapor pressure, if the temperature decreases, the saturated vapor pressure also decreases, so that the solvent vapor condenses and floating liquid drops are readily formed. Therefore, it is preferable that the solvent vapor be further heated after obtained in order that the temperature thereof does not decrease.
   As a concrete preparing method, for example, when the solvent is water, onto a metal plate heated to 120 °C, water is dropped 1 ml for two seconds at intervals of two minutes per 4 cm² of the metal plate. It is preferable that the solvent vapor be prepared in a hermetically sealed container. At this time, in order that the solvent vapor is not cooled, it is preferable that the temperature of the inner walls and the like of the container be maintained not less than the temperature of the solvent vapor.
(b) Floating liquid drops are absorbed from the solvent vapor containing floating liquid drops. This is performed by using the above-mentioned absorbent.

### Mixing of Carbon Dioxide and Solvent Vapor

To mix carbon dioxide containing no floating liquid drops and the solvent vapor containing no floating liquid drops, just mixing these two will do. It is preferable that these two be mixed in a hermetically sealed container. Moreover, since the solvent vapor forms floating liquid drops if the temperature of the mixture gas decreases after the mixing, it is preferable that these two be mixed while being heated. It is preferable that the heating temperature be not less than the temperature of the solvent vapor.

Specifically, it is preferable to set the heating temperature so that the temperature of the gas for transdermal absorption obtained after the heating is not more than 100 °C, and further, it is more preferable to set it so that the temperature is not more than 50 °C. However, it is preferable that the temperature of the gas for transdermal absorption obtained after the heating be not less than 25 °C. Moreover, it is preferable to heat also the inner wall of the mixing container itself. Further, it is preferable to prevent the temperature of the mixture gas from cooling by using a heat insulator for the outer wall of the mixing container.

### Structure Example of Apparatus

### First Preparing Apparatus

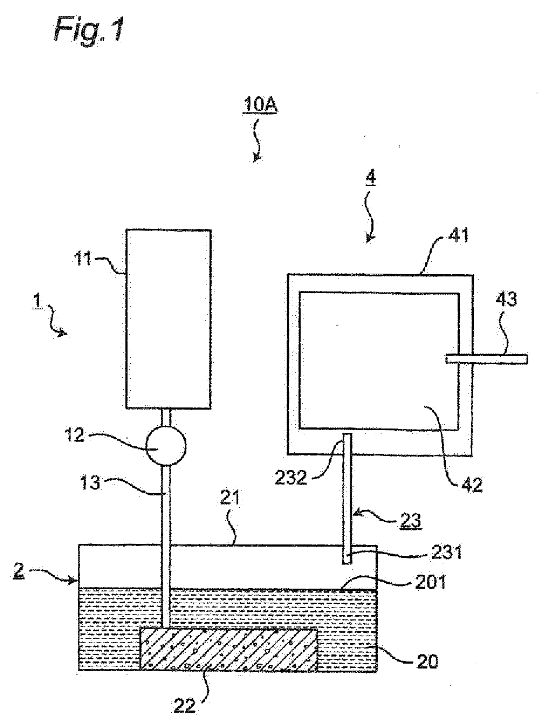

FIG. 1 is a schematic structure diagram showing an example of the first apparatus for preparing a gas for transdermal absorption of the present invention. This apparatus 10A is provided with a carbon dioxide supplying unit 1, a solvent vapor generating unit 2, and a floating liquid drop removing unit 4. The solvent vapor generating unit 2 serves also as a generating unit of a gas for transdermal absorption.

Specifically, the carbon dioxide supplying unit 1 is provided with a cylinder 11 storing liquefied carbon dioxide, a pressure regulator 12, and an outlet tube 13. The carbon dioxide supplying unit 1 supplies carbon dioxide from the cylinder 11 to the solvent vapor generating unit 2 through the outlet tube 13 after pressure-reducing it by the pressure regulator 12.

The solvent vapor generating unit r 2 is provided with a tank 21 storing a solvent 20, an air stone 22 placed in the tank 21, and an outlet tube 23. The tank 21 is a hermetically sealed container. The outlet tube 13 is coupled to the air stone 22. The quantity of the solvent 20 is set so that a liquid level 201 of the solvent 20 is situated above the air stone 22 and situated below an entrance end portion 231 of the outlet tube 23.

The floating liquid drop removing unit 4 is provided with a hermetically sealed container 41, a heater 42 placed in the hermetically sealed container 41, and an outlet tube 43. The outlet tube 23 communicates with the inside of the hermetically sealed container 41.

The apparatus 10A of the above-described structure operates as follows:
(1) The carbon dioxide supplying unit 1 operates to supply gaseous carbon dioxide to the air stone 22.
(2) From the air stone 22, fine bubbles of carbon dioxide are vigorously released into the solvent 20. Then, the bubbles reach the liquid level 201 of the solvent 20 and burst, whereby floating liquid drops of the solvent 20 are generated in the tank 21. On the other hand, since the solvent 20 is vaporable, the vapor of the solvent 20 is also generated in the tank 21.
(3) In the tank 21, the carbon dioxide and the vapor of the solvent 20 are mixed, and a gas for transdermal absorption containing a carbon dioxide-solvent complex formed by these two being combined is generated. This gas for transdermal absorption also contains the floating liquid drops of the solvent 20.
(4) The gas for transdermal absorption is supplied into the hermetically sealed container 41 by way of the outlet tube 23 by the gas pressure of the cylinder 11. Then, the gas for transdermal absorption is heated by the heater 42, whereby the floating liquid drops contained in the gas for transdermal absorption evaporate. As a result, a gas for transdermal absorption containing no floating liquid drops is obtained.
(5) The gas for transdermal absorption containing no floating liquid drops passes through the outlet tube 43 to be made available for use on the next process.

As described above, according to the apparatus 10A of the above-described structure, a gas for transdermal absorption containing no floating liquid drops can be obtained.

Although the solvent 20 in the tank 21 may be maintained at room temperature, it may be heated by a heater provided in the tank 21. By heating the solvent 20 in the tank 21, the evaporation of the floating liquid drops in the hermetically sealed container 41 of the floating liquid drop removing unit 4 occurs efficiently.

Moreover, the temperature of the heater 42 of the floating liquid drop removing unit 4 is preferably around the boiling point of the solvent 20 and is more preferably not less than the boiling point. However, if the temperature of the heater 42 is too high, the gas for transdermal absorption obtained from the outlet tube 43 is too hot and can burn the human body and the like. Therefore, it is preferable to set the temperature of the heater 42 so that the temperature of the obtained gas for transdermal absorption is 25 °C to 100 °C, preferably, not more than 50 °C. When the solvent 20 is, for example, water, it is preferable to set the temperature of the heater 42 to 100 °C to 150 °C. It is preferable that the temperature of the heater 42 be increased in advance.

Moreover, the checking of the generation of floating liquid drops in the tank 21 is performed by placing a moisture absorbing member such as filter paper at an exit end portion 232 of the outlet tube 23 and the moisture absorbing member getting wet.

Moreover, since the tank 21 is a hermetically sealed container, substantially all the air in the tank 21 is substituted by carbon dioxide after a predetermined time. However, it is not particularly necessary to set the concentration of the carbon dioxide supplied from the carbon dioxide supplying unit 1.

Further, the floating liquid drop removing unit 4 may serve also as a portion for dispensing the gas for transdermal absorption to an animal or a human. In that case, part of the hermetically sealed container 41 is formed of an opening and closing cover made of rubber or the like, the opening-closing cover is opened, a desired part of a human body or the like is placed in the hermetically sealed container 41, the opening-closing cover is closed, and the apparatus 10A is operated under that condition.

Or the gas for transdermal absorption having passed through the floating liquid drop removing unit 4 may be blown to a desired part of a human body or the like as it is. Or a desired part of a human body or the like is sealed by a sealing enclosure member, the gas for transdermal absorption having passed through the floating liquid drop removing unit 4 is filled into the sealed space as it is, and the desired part is exposed to the gas for transdermal absorption. The exposure time in that case is preferably not less than five minutes at each time, and is more preferably not less than ten minutes.

However, there is no limit to the exposure time. Since the gas for transdermal absorption having passed through the floating liquid drop removing unit 4 contains no floating liquid drops, it passes through the clothing or the like to be absorbed transdermally, and in that case, it wets neither the clothing or the like nor the skin. Needless to say, the gas for transdermal absorption may be used for a human body part not covered with clothing.

### Second Preparing Apparatus

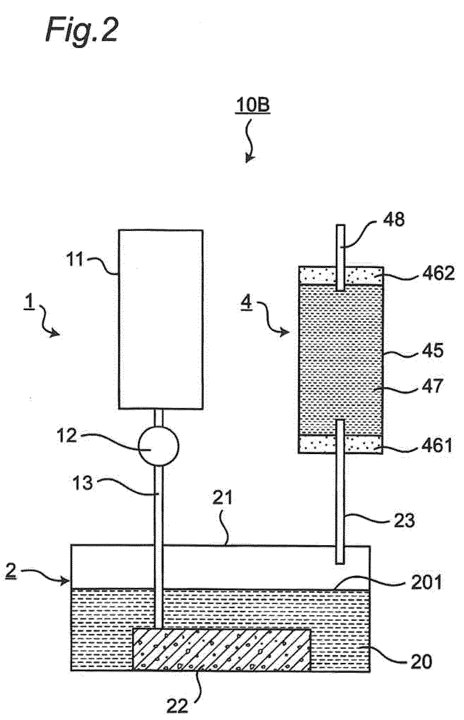

FIG. 2 is a schematic structure diagram showing another example of the first apparatus for preparing a gas for transdermal absorption of the present invention. This apparatus 10B is provided with the carbon dioxide supplying unit 1, the solvent vapor generating unit 2, and the floating liquid drop removing unit 4. The solvent vapor generating unit 2 serves also as a generating unit of a gas for transdermal absorption.

Specifically, the carbon dioxide supplying unit 1 and the solvent vapor generating unit 2 are the same as those of the apparatus 10A. The floating liquid drop removing unit 4 is provided with a tubular container 45, sponge members 461 and 462 closing both ends of the container 45, an absorbent 47 filled in the container 45, and an outlet tube 48. The outlet tube 23 passes through the sponge member 461 to communicate with the inside of the container 45. The outlet tube 48 passes through the sponge member 462.

The apparatus 10B of the above-described structure operates as follows:
(1) The carbon dioxide supplying unit 1 operates to supply gaseous carbon dioxide to the air stone 22.
(2) From the air stone 22, fine bubbles of carbon dioxide are vigorously released into the solvent 20. Then, the bubbles reach the liquid level 201 of the solvent 20 and burst, whereby floating liquid drops of the solvent 20 are generated in the tank 21. On the other hand, since the solvent 20 is vaporable, the vapor of the solvent 20 is also generated in the tank 21.
(3) In the tank 21, the carbon dioxide and the vapor of the solvent 20 are mixed, and a gas for transdermal absorption containing a carbon dioxide-solvent complex formed by these two being combined is generated. This gas for transdermal absorption also contains the generated floating liquid drops.
(4) The gas for transdermal absorption is supplied into the container 45 by way of the outlet tube 23 by the gas pressure of the cylinder 11. Then, the floating liquid drops contained in the gas for transdermal absorption are absorbed by the absorbent 47. As a result, a gas for transdermal absorption containing no floating liquid drops is obtained.
(5) The gas for transdermal absorption containing no floating liquid drops passes through the outlet tube 43 to be made available for use on the next process.

As described above, according to the apparatus 10B of the above-described structure, a gas for transdermal absorption containing no floating liquid drops can be obtained.

The absorbent 47 is a substance that does not chemically react with carbon dioxide or the solvent 20 and can absorb the floating liquid drops of the solvent 20. As the absorbent 47, the above-mentioned absorbent may be used.

The tubular container 45 preferably has a hermetically sealed structure, and any shape may be adopted such as a shape that is circular in transverse section or rectangular in transverse section.

The floating liquid drop removing unit 4 may have a heater that heats the gas for transdermal absorption in the container 45.

It is necessary to change the absorbent 47 filled in the container 45 to a new absorbent as appropriate since the absorption amount of the floating liquid drops is limited.

The rest is the same as in the case of the apparatus 10A.

### Third Preparing Apparatus

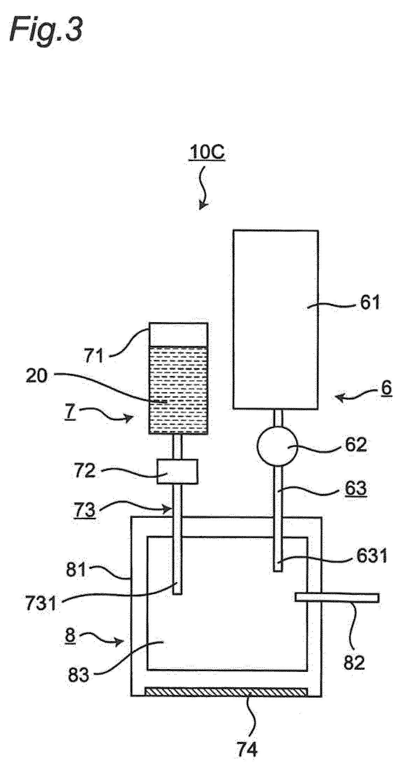

FIG. 3 is a schematic structure diagram showing an example of the second apparatus for preparing a gas for transdermal absorption of the present invention. This apparatus 10C is provided with a carbon dioxide supplying unit 6, a solvent vapor generating unit 7, and a generating unit 8 of a gas for transdermal absorption.

Specifically, the carbon dioxide supplying unit 6 is provided with a cylinder 61 storing liquefied carbon dioxide, a pressure regulator 62 and an outlet tube 63. The carbon dioxide supplying unit 6 supplies carbon dioxide from the cylinder 61 to the generating unit 8 of the gas for transdermal absorption through the outlet tube 63 after pressure-reducing it by the pressure regulator 62.

The solvent vapor generating unit 7 is provided with a tank 71 storing the solvent 20, an adjustment unit 72 that adjusts the mode of sending of the solvent 20 from the tank 71, an outlet tube 73, and a sheet heater 74 for vaporizing the solvent 20.

The generating unit 8 of the gas for transdermal absorption is provided with a hermetically sealed container 81 and an outlet tube 82. On the bottom surface in the container 81, the sheet heater 74 is installed, and above the sheet heater 74, an exit end portion 731 of the outlet tube 73 is situated. Moreover, in the container 81, an exit end portion 631 of the outlet tube 63 is also situated.

The apparatus 10C of the above-described structure operates as follows:
(1) The solvent vapor generating unit 7 operates. That is, the solvent 20 in the tank 71 is dropped onto the sheet heater 74 from the exit end portion 731 of the outlet tube 73. The temperature of the sheet heater 74 is previously increased to a temperature at which the solvent 20 vaporizes. The dropping mode of the solvent 20 is adjusted by the adjustment unit 72 so as to be a predetermined quantity per unit time at predetermined time intervals.
   Consequently, the dropped solvent 20 is instantaneously vaporized into vapor, so that no floating liquid drops are generated. Moreover, the temperature of the sheet heater 74 is set so that the vapor pressure of the solvent 20 in the container 81 does not exceed the saturated vapor pressure of the solvent 20 at the temperature of the sheet heater 74. That is, in the container 81, solvent vapor containing no floating liquid drops is generated.
(2) The carbon dioxide supplying unit 6 operates to supply gaseous carbon dioxide into the container 81 by way of the outlet tube 63. Since liquefied carbon dioxide is accommodated in the cylinder 61, the supplied carbon dioxide contains no floating liquid drops.
(3) Then, in the container 81, the carbon dioxide containing no floating liquid drops and the solvent vapor containing no floating liquid drops are mixed, and a gas for transdermal absorption containing a carbon dioxide-solvent complex formed by these two being combined is generated. Therefore, the generated gas for transdermal absorption contains no floating liquid drops.
(4) The gas for transdermal absorption containing no floating liquid drops passes through the outlet tube 82 to be made available for use on the next process.

As described above, according to the apparatus 10C of the above-described structure, a gas for transdermal absorption containing no floating liquid drops can be obtained.

In the hermetically sealed container 81 of the generating unit 8 of the gas for transdermal absorption, a heater 83 for heating gas may be provided. The temperature of the heater 83 is preferably not less than room temperature, particularly, not less than 40 °C. However, if the temperature of the heater 83 is too high, the gas for transdermal absorption obtained from the outlet tube 82 is too hot and can burn the human body and the like.

Therefore, it is preferable to set the temperature of the heater 83 so that the temperature of the obtained gas for transdermal absorption is 25 °C to 100 °C, preferably, not more than 50 °C. When the solvent 20 is, for example, water, it is preferable to set the temperature of the heater 83 to 100 °C to 150 °C. The temperature of the heater 83 is increased in advance.

As the sheet heater 74, for example, a common heater having a heating wire on the back surface of a heatproof plate made of a metal, ceramic or the like may be used. The temperature of the heater 74 is preferably around the boiling point of the solvent 20 and is more preferably not less than the boiling point. However, if the temperature of the heater 74 is too high, the gas for transdermal absorption obtained from the outlet tube 82 is too hot and can burn the human body and the like.

Therefore, it is preferable to set the temperature of the heater 74 so that the temperature of the obtained gas for transdermal absorption is 25 °C to 100 °C, preferably, not more than 50 °C. When the solvent 20 is, for example, water, it is preferable to set the temperature of the heater 74 to 100 °C to 150 °C.

A concrete example of the dropping mode of the solvent 20 is as follows: When the solvent 20 is water and the heater 74 is a metal plate set at 120 °C, the solvent 20 is dropped 1 ml for two seconds at intervals of one minute per 4 cm² of the heater 74.

It is preferable that carbon dioxide be supplied into the container 81 when the vapor pressure of the solvent 20 in the container 81 becomes not less than 30 % of the saturated vapor pressure of the solvent 20 at the temperature of the heater 74. For the vapor pressure of the solvent 20 in the container 81, although a higher vapor pressure is more preferable, it must not be higher than the saturated vapor pressure. This is because floating liquid drops of the solvent 20 are readily generated in the container 81 if the vapor pressure of the solvent 20 in the container 81 is higher than the saturated vapor pressure.

It is preferable that carbon dioxide be supplied into the container 81 so that the concentration in the container 81 be not less than 15 %, preferably, not less than 30 %. For the carbon dioxide concentration in the container 81, a higher concentration is more preferable, and there is no upper limit thereto.

While in the apparatus 10C, the generation of the solvent vapor and the mixing of the solvent vapor and carbon dioxide are performed in one container 81, they may be performed in different containers if no floating liquid drops are generated.

The container 81 may serve also as an administration unit of the gas for transdermal absorption to an animal or a human. In that case, part of the container 81 is formed of an opening-closing cover made of rubber or the like, the opening-closing cover is opened, a desired part of a human body or the like is placed in the container 81, the opening-closing cover is closed, and the apparatus 10C is operated under that condition.

### EXAMPLES

Next, the present invention will concretely be described with examples, but the present invention is not limited to these examples.

### Example 1

The present example relates to an example of the first method for preparing a gas for transdermal absorption.

### Preparing Apparatus

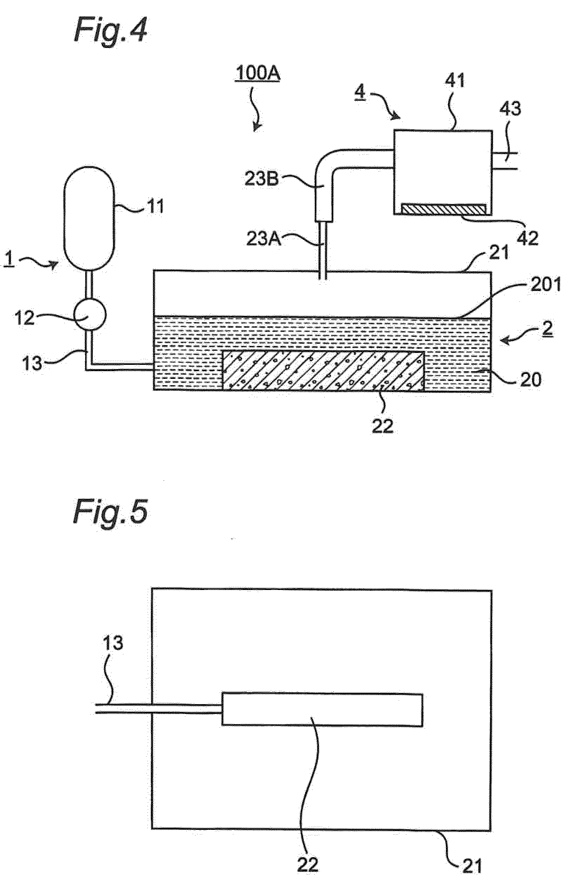

FIG. 4 is a side cross-sectional schematic diagram of a preparing apparatus used in the present example. FIG. 5 is a partially omitted plan view of the apparatus of FIG. 4. This apparatus 100A is provided with the carbon dioxide supplying unit 1, the solvent vapor generating unit 2, and the floating liquid drop removing unit 4. The solvent vapor generating unit 2 serves also as a generating unit of a gas for transdermal absorption.

The carbon dioxide supplying unit 1 is provided with the cylinder 11 storing liquefied carbon dioxide, the pressure regulator 12, and the outlet tube 13. The carbon dioxide supplying unit 1 supplies carbon dioxide from the cylinder 11 to the solvent vapor generating unit 2 through the outlet tube 13 after pressure-reducing it by the pressure regulator 12. The outlet tube 13 is a silicon outlet tube with an inside diameter of 5 mm.

The solvent vapor generating unit 2 is provided with the tank 21 storing the solvent 20, the air stone 22 placed in the tank 21, a joint 23A, and an outlet tube 23B. The tank 21 is made of polypropylene, has a rectangular parallelepiped shape, and has dimensions of 17 cm in length, 12 cm in width and 6 cm in height. The air stone 22 has a rectangular parallelepiped shape, is fixed to the bottom surface of the tank 21 by an adhesive agent, and has dimensions of 100 mm in length, 15 mm in width and 20 mm in height.

To the air stone 22, the outlet tube 13 passing through the side wall of the tank 21 is coupled. The joint 23A is fixed to the center of the upper surface of the tank 21, is made of plastic, and has dimensions of 25 mm in length and 2 mm in inside diameter.

The outlet tube 23B couples the joint 23A and the floating liquid drop removing unit 4 together, and is a silicon outlet tube with an inside diameter of 5 mm. The quantity of the solvent 20 is set so that the liquid level 201 of the solvent 20 is situated above the air stone 22 and situated below the end portion of the joint 23A.

The floating liquid drop removing unit 4 is provided with the hermetically sealed container 41, the heater 42 placed in the hermetically sealed container 41, and the outlet tube 43. The outlet tube 23B communicates with the inside of the hermetically sealed container 41.

### Operation Conditions

Solvent 20 ... deionized water 500 ml
Temperature of the heater 42 ... 105 °C
Carbon dioxide concentration in the container 41 ... 75 %
Heating time at the floating liquid drop removing unit 4 ... ten minutes

### Operation

The apparatus 100A operates as follows:
(1) The carbon dioxide supplying unit 1 operates to supply gaseous carbon dioxide to the air stone 22.
(2) From the air stone 22, fine bubbles of carbon dioxide are vigorously released into the solvent 20. Then, the bubbles reach the liquid surface 201 of the solvent 20 and burst, whereby floating liquid drops of the solvent 20 are generated in the tank 21. On the other hand, since the solvent 20 is vaporable, the vapor of the solvent 20 is also generated in the tank 21.
(3) In the tank 21, the carbon dioxide and the vapor of the solvent 20 are mixed, and a gas for transdermal absorption containing a carbon dioxide-solvent complex formed by these two being combined is generated. This gas for transdermal absorption also contains the floating liquid drops of the solvent 20.
(4) The gas for transdermal absorption is supplied into the hermetically sealed container 41 by way of the outlet tube 23B by the gas pressure of the cylinder 11. Then, the gas for transdermal absorption is heated by the heater 42, whereby the floating liquid drops contained in the gas for transdermal absorption evaporate. As a result, a gas for transdermal absorption containing no floating liquid drops is obtained. In the container 41, the temperature was 42 °C, and the humidity was 90 %.
(5) The gas for transdermal absorption containing no floating liquid drops passes through the outlet tube 43 to be made available for use on the next process.

As described above, according to the apparatus 100A, a gas for transdermal absorption containing no floating liquid drops can be obtained.

### Checking of Floating Liquid Drops

Although the inside of the container 41 was illuminated by a flashlight, no Tyndall phenomenon was recognized. Therefore, no floating liquid drops were present.

### Quantity Determination of Floating Liquid Drops

Performing measurement by the following method, the mass of the floating liquid drops was 0 mg.
(1) First, the total mass of filter paper with a diameter of 90 mm (JIS-P-3801 standard product) and a non-porous sealable aluminum pouch (11 cm in length, 11 cm in width) was measured to obtain a first measurement value.
(2) Then, after the filter paper was picked up with tweezers and exposed inside the container 41 for 30 seconds, it was immediately accommodated in the aluminum pouch in the container 41 and sealed completely. Then, the total mass thereof was measured to obtain a second measurement value.
(3) On the other hand, a third measurement value was obtained similarly to the above (2) under the same conditions as the temperature and humidity in the container 41.
(4) Then, the difference between the third measurement value and the first measurement value was subtracted from the difference between the second measurement value and the first measurement value to thereby obtain the mass of the floating liquid drops absorbed by the filter paper.

### Test Condition

A test to obtain the effect of the obtained gas for transdermal absorption was performed as follows: A male subject took off his socks, rolled up his trousers to the knees, sat on a chair, and put his both legs into an upward opening container. Then, a rubber sheet was placed so as to cover the legs and the upper opening of the container so that the container was half sealed. Then, the gas for transdermal absorption having passed through the outlet tube 43 was filled in the container, and this condition was maintained for five minutes. That is, the male subject's legs were exposed to the gas for transdermal absorption for five minutes.

### Effect Determination

The increase-decrease rate of the blood flow volume of the male subject's instep was obtained to thereby determine the effect. The measurement of the blood flow volume was performed by using a laser Doppler blood-flowmeter (product name "OMEGAZONE OZ-1" manufactured by OMEGAWAVE, INC.). First, the blood flow volume before the test was measured. Then, the male subject took out his legs from the container, and the blood flow volumes one minute later and two minutes later were measured. Table 1 shows the results.

**Table 1**

| | Temperature (°C) | Humidity (%) | Left leg | | Right leg | | Average blood flow volume | Average increase/ decrease % in blood flow volume |
|---|---|---|---|---|---|---|---|---|
| | | | Blood flow volume | Increase/ decrease % in blood flow volume | Blood flow volume | Increase/ decrease % in blood flow volume | | |
| Before test | 26 | 30 | 6.77 | - | 6.49 | | 6.63 | - |
| Example 1 (1 min. later) | 38 | 63 | 7.75 | 14.5 | 7.83 | 20.6 | 7.79 | 17.5 |
| Comparative Example 1 (1 min. later) | 26 | 56 | 6.92 | 2.2 | 6.46 | -0.5 | 6.69 | 0.9 |

### Comparative Example 1

In Comparative Example 1, although the apparatus 100A of Example 1 was used, it was used without the heater 42 energized. Moreover, the present comparative example was different from Example 1 only in the following point, and the rest was the same as in Example 1.

### Checking of Floating Liquid Drops

In the container 41, large liquid drops gushed out like a shower from the outlet tube 23B and dropped to the bottom of the container 41 in a short time to wet the bottom.

### Quantity Determination of Floating Liquid Drops

A filter paper with a diameter of 90 mm (JIS-P-3801 standard product) was picked up with tweezers, and exposed at a distance of 10 cm ahead of the outlet tube 23B for 30 seconds. The mass of the floating liquid drops was 41 mg.

### Effect Determination

The measurement of the blood flow volume after the test was performed only one minute later. Table 1 shows the results.

### Consideration

In Example 1, the blood flow volume one minute later increased 17.5 %, and the subject's legs did not get wet at all. On the contrary, in Comparative Example 1, although the blood flow volume of the left leg slightly increased, the blood flow volume of the right leg slightly decreased. This is considered to be a measurement accuracy error, and it is considered that there was no blood flow volume change in fact.

From the above, according to the gas for transdermal absorption obtained by Example 1, the action of carbon dioxide can be delivered powerfully.

### Example 2 to 10

The present examples relates to other examples of the first method for preparing a gas for transdermal absorption.

### Preparing Apparatus

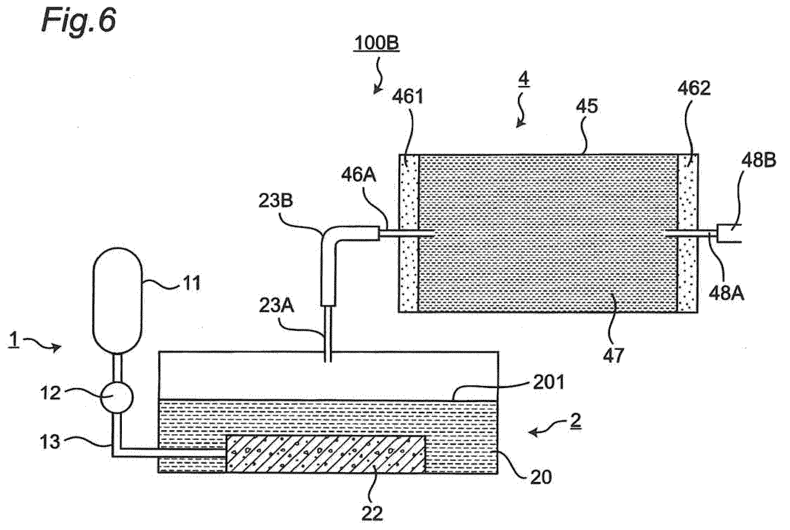

FIG. 6 is a side cross-sectional schematic diagram of a preparing apparatus used in the present example. This apparatus 100B is provided with the carbon dioxide supplying unit 1, the solvent vapor generating unit 2, and the floating liquid drop removing unit 4. The solvent vapor generating unit 2 serves also as a generating unit of a gas for transdermal absorption.

The carbon dioxide supplying unit 1 and the solvent vapor generating unit 2 are the same as those of the apparatus 100A. The floating liquid drop removing unit 4 is provided with the tubular container 45, the sponge members 461 and 462 closing both ends of the container 45, the absorbent 47 filled in the container 45, a joint 46A, a joint 48A, and an outlet tube 48B.

The outlet tube 23B communicates with the inside of the container 45 through the joint 46A passing through the center of the sponge member 461. The outlet tube 48B is coupled to the joint 48A passing through the center of the sponge member 462. The tubular container 45 is made of plastic, is circular in transverse section, and has dimensions of 15 cm in length and 8 cm in diameter. The sponge members 461 and 462 are synthetic resin foams, and have dimensions of 1 cm in thickness and 8 cm in diameter.

### Operation Conditions

The kind and temperature of the solvent 20, the kind of the absorbent 47, the carbon dioxide concentration in the container 45 are as shown in Table 2.

**Table 2**

| | Solvent | Liquid temperature | pH adjuster | pH | Floating liquid drop removing unit | Humidity (%) before floating liquid drop removal | Humidity(%) after floating liquid drop removal | CO2 concentration (%) | Gas for percutaneous absorption: exposure time (min.) | Blood flow volume increase rate (%) |
|---|---|---|---|---|---|---|---|---|---|---|
| Example 2 | Water | 26 | - | 4.6 | Calcium chloride | 75 | 30 | 99 | 3 | 48.3 |
| | | | | | | | | | 15 | 58.0 |
| Example 3 | Water | 26 | Phosphate buffer solution | 7.2 | Calcium chloride | 73 | 32 | 99 | 3 | 34.7 |
| | | | | | | | | | 15 | 57.9 |
| Example 4 | Water | 26 | Phosphate buffer solution | 7.2 | Zeolite | 75 | 62 | 99 | 5 | 21.5 |
| | | | | | | | | | 15 | 58.9 |
| Example 5 | Water | 26 | Citric acid | 2.5 | Calcium chloride | 88 | 37 | 96 | 3 | 20.4 |
| Example 6 | Water | 26 | - | 4.6 | Silica gel | 75 | 31 | 99 | 3 | 42.7 |
| Example 7 | Water | 26 | - | 4.6 | Zeolite | 75 | 62 | 99 | 5 | 21.5 |
| | | | | | | | | | 15 | 58.9 |
| Example 8 | Water | 60 | - | 4.6 | Calcium chloride | 99 | 50 | 99 | 3 | 28.8 |
| Example 9 | Isopropanol | 26 | - | - | Zeolite | - | - | 99 | 3 | 64.2 |
| Example 10 | Jojoba oil | 26 | - | - | Zeolite | - | - | 99 | 3 | 24.0 |
| Comparative Example 2 | Water | 26 | - | 4.6 | - | 75 | - | 99 | 3 | 1.6 |
| Comparative Example 3 | Water | 60 | - | 4.6 | - | 99 | - | 99 | 3 | 19.6 |
| Comparative Example 4 | Isopropanol | 26 | - | - | - | - | - | 97 | 3 | 4.9 |
| Comparative Example 5 | Jojoba oil | 26 | - | - | - | - | - | 99 | 3 | -1.9 |

### Operation

The apparatus 100B operates as follows:
(1) The carbon dioxide supplying unit 1 operates to supply gaseous carbon dioxide to the air stone 22.
(2) From the air stone 22, fine bubbles of carbon dioxide are vigorously released into the solvent 20. Then, the bubbles reach the liquid surface 201 of the solvent 20 and burst, whereby floating liquid drops of the solvent 20 are generated in the tank 21. On the other hand, since the solvent 20 is vaporable, the vapor of the solvent 20 is also generated in the tank 21.
(3) In the tank 21, the carbon dioxide and the vapor of the solvent 20 are mixed, and a gas for transdermal absorption containing a carbon dioxide-solvent complex formed by these two being combined is generated. This gas for transdermal absorption also contains the floating liquid drops of the solvent 20.
(4) The gas for transdermal absorption is supplied into the container 45 by way of the outlet tube 23B by the gas pressure of the cylinder 11. The gas pressure is the same as that of Example 1. Then, the floating liquid drops contained in the gas for transdermal absorption are absorbed by the absorbent 47. As a result, a gas for transdermal absorption containing no floating liquid drops is obtained.
(5) The gas for transdermal absorption containing no floating liquid drops passes through the outlet tube 48B to be made available for use on the next process.

As described above, according to the apparatus 100B, a gas for transdermal absorption containing no floating liquid drops can be obtained.

### Determination of Floating Liquid Drop Quantity

The gas for transdermal absorption having passed through the outlet tube 48B was directed into a plastic bag having a check valve at the bottom, through the check valve. The plastic bag has dimensions of 80 cm in length and 20 cm in width. Then, filter paper with a diameter of 90 mm (JIS-P-3801 standard product) was picked up with tweezers and exposed at the bottom in the plastic bag for 30 seconds. The mass of the floating liquid drops was 8 mg in Example 4, 7 mg in Example 7, and 0 mg in the other examples.

### Test Condition 1

A male subject's left arm was covered with a plastic bag having a check valve at the bottom. The plastic bag has dimensions of 80 cm in length and 20 cm in width. Then, the gas for transdermal absorption having passed through the outlet tube 48B was directed into the plastic bag through the check valve. Then, the left arm was exposed for a predetermined time. Thereafter, the blood flow volume of the left arm was measured by using the above-mentioned laser Doppler blood-flowmeter, and was compared with the blood flow volume measured before the exposure. Table 2 shows the results.

### Test Condition 2

The subject was a 47-year-old woman having been suffering from leg coldness for many years. The subject's right leg with a stocking on was put in a polypropylene bag having a check valve. The bag has dimensions of 80 cm in length and 20 cm in width. Then, on the knee of the subject's right leg, the top of the bag was tied with a textile belt to hermetically seal the bag. Then, the gas for transdermal absorption obtained in Example 2 was filled into the bag through the check valve. Then, the right leg was exposed for ten minutes.

Approximately one minute after the filling, the subject felt very strong warmness in the entire right leg; however, the stocking did not get wet at all. The subject felt warmness in the right leg for several hours also after the bag was removed. The subject did not feel any improvement on the coldness of the left leg. In this manner, the subject continued the exposure to the gas for transdermal absorption for ten minutes once a day for five days. As a result, the coldness in the subject's right leg was gone and the leg coldness was improved.

### Comparative Examples 2 to 5

In the present comparative examples, an apparatus not provided with the floating liquid drop removing unit 4 compared with the apparatus 100B was used. Moreover, the present comparative examples were different from Examples 2 to 10 only in the following point, and the rest was the same as in Examples 2 to 10.

### Operation Conditions

The kind and temperature of the solvent 20, the kind of the absorbent 47, the carbon dioxide concentration in the container 45 are as shown in Table 2.

### Quantity Determination of Floating Liquid Drops

Measurement was performed similarly to in Examples 2 to 10. The mass of the floating liquid drops was 42 mg in Comparative Example 2 and 46 mg in Comparative Example 3.

### Test Condition 1

The gas for transdermal absorption having passed through the outlet tube 23B was directed into a plastic bag through a check valve. The rest was performed similarly to in the case of Examples 2 to 10. Table 2 shows the results.

### Example 11

The present example relates to the second method for preparing a gas for percutaneous transdermal absorption.

### Preparing Apparatus

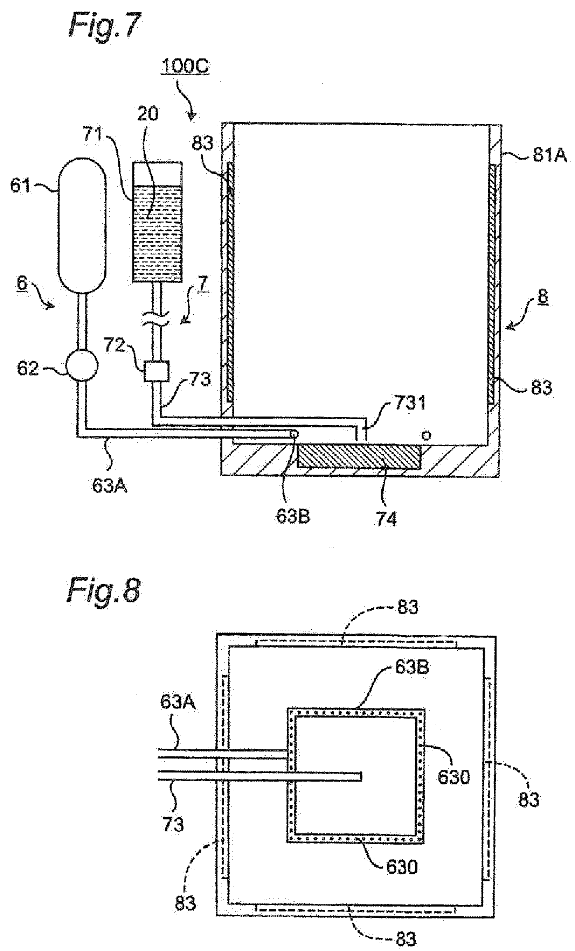

FIG. 7 is a side cross-sectional schematic diagram of a preparing apparatus used in the present example. FIG. 8 is a partially omitted plan view of the apparatus of FIG. 7. This apparatus 100C is provided with the carbon dioxide supplying unit 6, the solvent vapor generating unit 7, and a generating unit 8 of a gas for transdermal absorption.

The carbon dioxide supplying unit 6 is provided with a cylinder 61 storing liquefied carbon dioxide, a pressure regulator 62, an outlet tube 63A, and a jetting unit 63B. The outlet tube 63A is coupled to the jetting unit 63B. The carbon dioxide supplying unit 6 supplies carbon dioxide from the cylinder 61 to the generating unit 8 of the gas for transdermal absorption through the outlet tube 63A and the jetting unit 63B after pressure-reducing it by the pressure regulator 62.

The outlet tube 63A is a brass pipe with an inside diameter of 3 mm, and extends through the side wall of a box 81A of the generating unit 8 of the gas for transdermal absorption. The jetting unit 63B is structured so that the brass pipe with an inside diameter of 3 mm is circularly disposed so as to surround the sheet heater 74 of the solvent vapor generating unit 7. The jetting unit 63B is square when viewed two-dimensionally, and is 17 cm on a side. Moreover, on the jetting unit 63B, upward jetting nozzles 630 with a diameter of 1 mm are formed at intervals of 1 cm.

The solvent vapor generating unit 7 is provided with the tank 71 storing the solvent 20, the adjustment unit 72 that adjusts the mode of supply of the solvent 20 from the tank 71, the outlet tube 73, and the sheet heater 74 for vaporizing the solvent 20. The adjustment unit 72 has a motor-operated valve whose opening-closing time and opening-closing interval can be adjusted automatically.

The sheet heater 74 is placed at a depth of 3 cm from the bottom surface in the center of the bottom surface of the box 81A of the generating unit 8 of the gas for transdermal absorption, is square when viewed two-dimensionally, and is 15 cm on a side. The outlet tube 73 which is a stainless-steel pipe with an inside diameter of 3 mm extends to above the center of the sheet heater 74 and bends 90 degrees downward.

The generating unit 8 of the gas for transdermal absorption has the upward opening box 81A. The box 81A is made of stainless steel, is square when viewed two-dimensionally, is 40 cm in height, and is 35 cm on a side. The upper opening of the box 81A is covered with a rubber cover. Moreover, the sheet heaters 83 are provided on the four side walls of the box 81A.

### Operation Conditions

Solvent 20 ... deionized water
Temperature of the sheet heater 74 ... 105 °C
Mode of supply of the solvent 20 from the outlet tube 73 ... intermittently dropped 1 ml every 30 seconds
Temperature of the sheet heater 83 ... 50 °C
Carbon dioxide concentration in the box 81A... 75 %

### Operation

The apparatus 100C operates as follows:
(1) The solvent vapor generating unit 7 operates. That is, the solvent 20 in the tank 71 is dropped onto the sheet heater 74 from the exit end portion 731 of the outlet tube 73. The temperature of the sheet heater 74 is increased to 105 degree C in advance. The dropping mode of the solvent 20 is adjusted by the adjustment unit 72 so that the solvent 20 is intermittently dropped 1 ml every 30 seconds.
   Consequently, the dropped solvent 20 is instantaneously vaporized into vapor, so that no floating liquid drops are generated. Moreover, the temperature of the sheet heater 74 is set so that the vapor pressure of the solvent 20 in the box 81A does not exceed the saturated vapor pressure of the solvent 20 at the temperature of the sheet heater 74. That is, in the box 81A, solvent vapor containing no floating liquid drops is generated.
(2) The carbon dioxide supplier 6 operates to supply gaseous carbon dioxide into the box 81 A. Since liquefied carbon dioxide is stored in the cylinder 61, the supplied carbon dioxide contains no floating liquid drops.
(3) Then, in the box 81A, the carbon dioxide containing no floating liquid drops and the solvent vapor containing no floating liquid drops are mixed, and a gas for transdermal absorption containing a carbon dioxide-solvent complex formed by these two being combined is generated. Therefore, the generated gas for transdermal absorption contains no floating liquid drops.

As described above, according to the apparatus 100C, a gas for transdermal absorption containing no floating liquid drops can be obtained.

### (Quantity Determination of Floating Liquid Drops)

Picking up a filter paper with tweezers, exposing it in the generator 8 of the gas for transdermal absorption for 30 seconds and performing measurement in a similar manner to that of Example 1, the mass of the floating liquid drops was 0 mg.

### Test Condition

The same as in Example 1.

### Effect Determination

The same as in Example 1.

### Comparative Example 6

While the apparatus 100C was used, the solvent 20 was not supplied from the tank 71 but 100 ml of solvent 20 was put in the box 81A. Consequently, floating liquid drops were generated. The rest was the same as in Example 11 except for the following point:

### Operation Conditions

Solvent 20 ... deionized water
Temperature of the sheet heater 74 ... 105 °C
Mode of supply of the solvent 20 from the outlet tube 73 ... water was not supplied
Temperature of the heater 83 ... 50 °C
Carbon dioxide concentration in the box 81A... 75 %

### Checking of Floating Liquid Drops

In the box 81A, a lot of white misty floating liquid drops were recognized.

### (Quantity Determination of Floating Liquid Drops)

Performing measurement by the method of Example 1, the mass of the floating liquid drops was 33 mg.

### Test Condition

The same as in Example 1.

### (Effect Determination)

The same as in Example 1.

### Comparative Example 7

While the apparatus 100C was used, carbon dioxide was not supplied. The rest was the same as in Example 11 except for the following point:

### Operation Conditions

Temperature of the sheet heater 74 ... 105 °C
Mode of supply of the solvent 20 from the outlet tube 73 ... intermittently dropped 1 ml every 30 seconds
Temperature of the heater 83 ... 50 °C
Carbon dioxide concentration in the box 81A... 0%

### Checking of Floating Liquid Drops

Although the inside of the box 81A was illuminated by a flashlight, no Tyndall phenomenon was recognized.

### Quantity Determination of Floating Liquid Drops

Performing measurement by the method of Example 1, the mass of the floating liquid drops was 0 mg.

### (Test Condition

The same as in Example 1.

### Effect Determination

The same as in Example 1.

### Results

The results of Example 11 and Comparative Examples 6 and 7 are shown in Table 3.

**Table 3**

| | Temperature (°C) | Humidity (%) | Left leg | | Right leg | | Average blood flow volume | Average increase/ decrease % in blood flow volume |
|---|---|---|---|---|---|---|---|---|
| | | | Blood flow volume | Increase/ decrease % in blood flow volume | Blood flow volume | Increase/ decrease % in blood flow volume | | |
| Before test | 26 | 61 | 6.71 | - | 6.49 | - | 6.60 | |
| Example 11 (1 | 38 | 70 | 9.69 | 44.4 | 9.12 | 40.5 | 9.41 | 42.6 |
| Example 11 (2 min. later) | | | 9.33 | 39.0 | 8.75 | 34.8 | 9.04 | 37.0 |
| Comparative Example 6 (1 min. later) | 45 | 95 | 8.55 | 27.4 | 7.89 | 21.6 | 8.22 | 24.5 |
| Comparative Example 6 (2 min. later) | | | 6.59 | -1.8 | 6.40 | -1.4 | 6.50 | -1.6 |
| Comparative Example 7 (1 min. later) | 38 | 77 | 9.29 | 38.5 | 8.52 | 31.3 | 8.91 | 34.9 |
| Comparative Example 7 (2 min. later) | | | 7.73 | 15.2 | 8.01 | 23.4 | 7.87 | 19.2 |

### Discussion

In Example 11, the average blood flow volume increase rate was 42.6 % one minute later, and was 37.0 % two minutes later. Thus, Example 11 not only had a strong blood flow volume increasing effect but also was excellent in the persistence of the blood flow volume increasing action. In addition, the subject's legs did not get wet at all.

In Comparative Example 6, a large amount of floating liquid drops were generated, a volume of condensation was recognized on the inner wall of the box 81A and the like, the humidity was 95 %, and the temperature reached 45 °C. The subject's legs were entirely wet because of steam, and although feeling an oppressive heat, the subject endured it. The average blood flow volume increase rate was no higher than 24.5 % one minute later. Moreover, while it exhibited -1.6 % two minutes later, this was conjectured to be a rebound phenomenon after the rapid increase in skin temperature due to the high-temperature floating liquid drops.

In Comparative Example 7, only heated water vapor was generated and no floating liquid drops were generated. The temperature in the box 81A increased to 38 °C. Consequently, while the average blood flow volume increase rate was 36.8 % one minute later, it was no higher than 19.2 % two minutes later. The subject's legs did not get wet at all.

From the above results, in Example 11, the effect of transdermal absorption of carbon dioxide can be delivered powerfully, and further, it can be delivered with excellent continuousness.

### Example 12

The carbon dioxide concentration in the box 81A was 30 %, and the rest was the same as in Example 11.

### Checking of Floating Liquid Drops

Although the inside of the box 81A was illuminated by a flashlight, no Tyndall phenomenon was recognized.

### Quantity Determination of Floating Liquid Drops

Performing measurement by the method of Example 1, the mass of the floating liquid drops was 0 mg.

### Results

The results of Example 12 are shown in Table 4.

**Table 4**

| | Temperature (°C) | Humidity (%) | Left leg | | Right leg | | Average blood flow volume | Average increase/ decrease % in blood flow volume |
|---|---|---|---|---|---|---|---|---|
| | | | Blood flow volume | Increase/ decrease % in blood flow volume | Blood flow volume | Increase/ decrease % in blood flow volume | | |
| Before test | 26 | 56 | 6.71 | - | 6.49 | - | 6.60 | - |
| Example 12 (1 min. later) | 38 | 77 | 10.27 | 47.1 | 9.08 | 36.2 | 9.68 | 42.1 |
| Example 12 (2 min. later) | | | 9.20 | 31.8 | 8.39 | 26.5 | 8.80 | 29.2 |

### Discussion

In Example 12, although the carbon dioxide concentration was no more than 30 %, the average blood flow volume increase rate was 42.1 % one minute later and 29.2 % two minutes later. Therefore, in Example 12, the effect of transdermal absorption of carbon dioxide can be delivered powerfully, and further, it can be delivered with excellent continuousness.

### INDUSTRIAL APPLICABILITY

The apparatus for preparing a gas for transdermal absorption of the present invention from which a gas for transdermal absorption containing no floating liquid drops of the solvent can be obtained is highly useful in industry.

### EXPLANATION OF REFERENCE NUMERALS

- 1: carbon dioxide supplying unit
- 2: solvent vapor generating unit
- 4: floating liquid drop removing unit
- 6: carbon dioxide supplying unit;
- 2: solvent vapor generating unit;
- 7: solvent vapor generating unit

- 8: generating unit of a gas for transdermal absorption
- 42: heater (heating means);
- 47: absorbent (absorbing means);
- 74: sheet heater (heating means);

## Claims

1. An apparatus for preparing a gas for transdermal absorption containing a gaseous carbon dioxide-solvent complex, comprising:
- a carbon dioxide supplying unit that is adapted to supply gaseous carbon dioxide;
- a solvent vapor generating unit that is adapted to generate vapor of a solvent, being capable of dissolving carbon dioxide and vaporable; and
- a generating unit of a gas for transdermal absorption that is adapted to generate the gas for transdermal absorption containing the gaseous carbon dioxide-solvent complex by mixing the supplied carbon dioxide and the generated solvent vapor; and
(a) further comprising a floating liquid drop removing unit that is adapted to remove a floating liquid drop of the solvent from the generated gas for transdermal absorption, or
(b) including a contamination preventing means for preventing contamination of the floating liquid drop of the solvent into the generated gas for transdermal absorption.

2. The apparatus for preparing the gas for transdermal absorption according to claim 1,
wherein the floating liquid drop removing unit has heating means for evaporating the floating liquid drop contained in the gas for transdermal absorption.

3. The apparatus for preparing the gas for transdermal absorption according to claim 1,
wherein the floating liquid drop removing unit has absorbing means for absorbing the floating liquid drop contained in the gas for transdermal absorption.

4. The apparatus for preparing the gas for transdermal absorption according to claim 3,
wherein the absorbing means is a chemical absorbent or a physical absorbent.

5. The apparatus for preparing the gas for transdermal absorption according to claim 1,
wherein the contamination preventing means is the carbon dioxide supplying unit configured so as to supply the carbon dioxide containing no floating liquid drop and the solvent vapor generating unit is configured so as to generate the solvent vapor containing no floating liquid drop.

6. The apparatus for preparing the gas for transdermal absorption according to claim 5,
wherein the carbon dioxide supplying unit is configured so as to supply liquefied carbon dioxide after vaporizing it.

7. The apparatus for preparing the gas for transdermal absorption according to claim 5,
wherein the carbon dioxide supplying unit has heating means for evaporating the floating liquid drop contained in the carbon dioxide.

8. The apparatus for preparing the gas for transdermal absorption according to claim 5,
wherein the carbon dioxide supplying unit has absorbing means for absorbing the floating liquid drop contained in the carbon dioxide.

9. The apparatus for preparing the gas for transdermal absorption according to claim 5,
wherein the solvent vapor generating unit has heating means for heating the solvent so that a vapor pressure thereof is not more than the saturated vapor pressure at that temperature.

10. The apparatus for preparing the gas for transdermal absorption according to claim 5,
wherein the solvent vapor generating unit has absorbing means for absorbing the floating liquid drop contained in the solvent vapor.

11. A method for preparing a gas for transdermal absorption containing a gaseous carbon dioxide-solvent complex, comprising:
- a carbon dioxide supplying process for supplying gaseous carbon dioxide;
- a solvent vapor generating process for generating vapor of a solvent, being capable of dissolving the carbon dioxide and vaporable; and
- a gas for transdermal absorption generating process for generating a gas for transdermal absorption containing the gaseous carbon dioxide-solvent complex by mixing the supplied carbon dioxide and the generated solvent vapor; and
(a) further comprising a floating liquid drop removing process for removing a floating liquid drop of the solvent from the generated gas for transdermal absorption, or
(b) including a contamination preventing process for preventing contamination of the floating liquid drop of the solvent into the generated gas for transdermal absorption.

12. The method for preparing the gas for transdermal absorption according to claim 11,
wherein the floating liquid drop removing process includes a heating process for evaporating the floating liquid drop contained in the gas for transdermal absorption.

13. The method for preparing the gas for transdermal absorption according to claim 11,
wherein the floating liquid drop removing process includes a absorbing process for absorbing the floating liquid drop contained in the gas for transdermal absorption.

14. The method for preparing the gas for transdermal absorption according to claim 13,
wherein the absorbing process is performed by using a chemical absorbent or a physical absorbent.

15. The method for preparing the gas for transdermal absorption according to claim 11,
wherein the contamination preventing process is the carbon dioxide supplying process for supplying the carbon dioxide containing no floating liquid drop and the solvent vapor generating process for generating the solvent vapor containing no floating liquid drop.

16. The method for preparing the gas for transdermal absorption according to claim 15,
wherein the carbon dioxide supplying process supplies liquefied carbon dioxide.

17. The method for preparing the gas for transdermal absorption according to claim 15,
wherein the carbon dioxide supplying process includes a heating process for evaporating the floating liquid drop contained in the carbon dioxide.

18. The method for preparing the gas for transdermal absorption according to claim 15,
wherein the carbon dioxide supplying process includes a absorbing process for absorbing the floating liquid drop contained in the carbon dioxide.

19. The method for preparing the gas for transdermal absorption according to claim 15,
wherein the solvent vapor generating process includes a heating process for heating the solvent so that the vapor pressure thereof is not more than the saturated vapor pressure at that temperature.

20. The method for preparing the gas for transdermal absorption according to claim 15,
wherein the solvent vapor generating process includes a absorbing process for absorbing the floating liquid drop contained in the solvent vapor.

21. A gas for transdermal absorption **characterized by**
being obtained by using the apparatus for preparing a gas for transdermal absorption according to any one of claims 1 to 10.

22. A gas for transdermal absorption **characterized by**
being obtained by the method for preparing a gas for transdermal absorption according to any one of claims 11 to 20.
